# EUROPEAN PATENT SPECIFICATION

(11) **EP 2 874 610 B1**
(45) Date of publication and mention of the grant of the patent: **29.08.2018**
(21) Application number: 13739675.0
(22) Date of filing: 18.07.2013
(51) Int. Cl.: A61K 9/00, A61K 9/20, A61K 9/24, A61K 45/06, A61K 31/192, A61K 31/195, A61K 9/46

(54) **MULTILAYERED PHARMACEUTICAL FORMULATION**
MEHRSCHICHTIGE PHARMAZEUTISCHE FORMULIERUNG
FORMULATION PHARMACEUTIQUE MULTICOUCHE

(30) Priority: 19.07.2012 IT MI20121263
(43) Date of publication of application: 27.05.2015
(73) Proprietor: Recordati Industria Chimica E Farmaceutica SPA, 20148 Milano (IT)
(72) Inventor: COLOMBO, Paolo, 43121 Parma (IT); ROSSI, Alessandra, 43121 Parma (IT); SONVICO, Fabio, 22071 Cadorago (CO) (IT); BARCHIELLI, Marco, 20020 Arese (MI) (IT)
(74) Representative: Longoni, Alessandra
(86) International application number: PCT/EP2013/065167
(87) International publication number: WO 2014/013006

(56) References cited:
- WO-A1-95/01781
- WO-A1-99/17745
- WO-A2-2011/077239
- WO-A2-2011/124953

## Description

The present invention relates to an oral trilayered formulation with multi-kinetic release in different sites of the gastro-intestinal tract, particularly suitable for the administration of combinations of active ingredients.

The trilayered technology has been already used in sustained release preparations for oral administration. In the patent literature there are several trilayered systems able to release one or more drugs from the different layers at different rates.

US 4,839,177 describes a controlled release system consisting of three layers wherein the external layers consist of an insoluble drug-free polymeric material, while the central layer is a swellable polymeric matrix containing the active ingredient. A linear release profile is obtained by coating the intermediate layer. In this case only one layer contains the drug while the other two layers are functional to the release modulation.

In other patent documents the layers contain one or more drugs and each layer is formulated in a different way; for example, in WO94/06416 a layer promptly releases one or more drugs, a second layer releases in a slow controlled way the same drug or the same drug combination present in the disintegrating layer and a third layer having barrier properties and with low permeability can be placed between the layers or over one of them. In this case the layers have only a time control on the release and cannot limit such release in the stomach for long time or limit the release in the intestine for one of the two drugs.

In WO95/01781 there are three layers containing the drug: the first layer releases a portion of the total dose of the drug, while the second one releases another portion of the drug in a controlled manner and the third layer releases the last portion of the drug more slowly than the second layer. Also in this case there is a time release control only and there is no spatial control as well as no control on the release position in the gastro-intestinal tract.

WO99/17745 describes a trilayered system consisting of a disintegrating layer (layer D), a layer consisting of an erodible matrix (layer E) and a controlled release swellable layer (layer R). Each layer contains one or more active ingredients which release rate changes depending from the relative position of the single layers in the trilayered tablet. In particular, when the erodible layer is the central layer (RED system), there is a fast initial release followed by a slower and almost linear release of the active ingredients. When the swellable layer is the central layer (DRE system), an anomalous Fick kinetics with no biphasic release is achieved. When the central layer is the disintegrating layer (RDE system), a linear release of the active ingredients is achieved.

In the trilayered systems described in the literature the release rate is modulated but there is no spatial control, that is a control on the release site allowing also the modulation of the absorption of the active ingredients in different tracts of the gastro-intestinal system.

We have now found an oral trilayered formulation with multi-kinetic release and spatial control which allows to achieve different release kinetics and release sites for each active ingredient contained in it. This oral trilayered formulation is particularly advantageous for the administration of combinations of active ingredients with pharmacodynamic properties and/or dosage schedules different each other but with complementary therapeutic effect.

Therefore, object of the present invention is a trilayered tablet for oral administration comprising a rapid disintegrating central layer (layer B) interposed between a layer with a swellable floating sustained release hydrophilic matrix (layer A) and a layer with a gastro-resistant or sustained release or gastro-resistant sustained release hydrophilic matrix (layer C).

The tablet according to the invention, even if contains a trilayered system analogous to the RDE system described in WO99/17745, is completely different from it for the multi-kinetic release and for the specificity of the release sites.

Due to the specific combination of the layers ABC, the tablet according to the present invention shows the following spatial-time release pattern which is its characterizing and innovative aspect:
- layer A is formulated for a sustained release under floating conditions and consists of a hydrophilic matrix which swells and floats in an aqueous medium releasing the drug in a sustained way in the first tract of the gastro-intestinal apparatus, preferably in the stomach.;
- layer B is formulated to rapidly disintegrate so rapidly and immediately releasing the drug optionally contained in it in the stomach and causing the split of the multilayered tablet into the two portions corresponding to layers A and C, which continue their transit and the release of the drugs in the gastro-intestinal tract independently from each other;
- layer C is a gastro-resistant or a sustained release or a controlled release gastro-resistant formulation mainly releasing the drug in the intestine. Preferably layer C is a controlled release gastro-resistant formulation releasing the drug in the intestine for a period of some hours.

In the present contest, unless otherwise specified, drug contained in a layer of the tablet of the invention means the active ingredient or the combination of active ingredients present in the layer.

Each layer of the tablet according to the invention can contain the same active ingredient or the same combination of active ingredients with respect to one or both the other layers or can contain an active ingredient or a combination of active ingredients different from the other two layers.

Furthermore layer B can also be drug-free and exclusively act as disintegrating layer which allows the split of the tablet of the invention into the two portions corresponding to layers A and C.

Preferably, at least a layer of the tablet of the invention contains a drug different from the other two layers.

The tablet of the invention is particularly suitable for the administration of a combination of two active ingredients with pharmacokinetic characteristics and dosage schedule different from each other which usually are administered to the patient in different forms and at different times.

The tablet object of the present invention allows to administer the two active ingredients in a single pharmaceutical dosage form, achieving different profiles of modified release synchronized each other, so increasing the compliance of the patient and decreasing the risk of mistakes in the administration of the drugs.

Specific examples of active ingredients which can be used in combination in the tablets object of the present invention are those active ingredients having a higher absorption in the proximal intestinal tract, such as gabapentin (an A2d ligand), ACE-inhibitors (for example captopril, lisinopril, enalapril, ramipril), metformin, baclofen, or having their action site at gastric level, such as H2-inhibitors (for example ranitidine), antibiotics used for Helicobacter Pylori eradication (for example amoxicillin, clarithromycin, metronidazole) together with products which need gastroprotection, such as proton pump inhibitors or non steroidal anti-inflammatory agents (NSAIDs), or need a sustained release in the intestine, such as calcium antagonists.

Preferred combinations within the scope of the present invention are combinations of proton pump inhibitors (for example omeprazole, esomeprazole, lansoprazole, pantoprazole) and H2 receptor antagonists (cimetidine, ranitidine, famotidine); NSAIDs (for example flurbiprofen, ibuprofen, piroxicam, meloxicam, diclofenac, naproxen) and proton pump inhibitors or H2 receptor antagonists (for example cimetidine, ranitidine); ACE-inhibitors (for example captopril, enalapril, ramipril, lisinopril) and angiotensin II receptor antagonists (for example valsartan, losartan, eprosartan, candesartan, irbesartan, olmesartan, telmisartan); ACE-inhibitors and calcium antagonists (for example amlodipine, nifedipine, lercanidipine, felodipine, diltiazem, verapamil); angiotensin II receptor antagonists and calcium antagonists; antibiotics used for Helicobacter Pylori eradication (for example amoxicillin, clarythromycin, metronidazole) and proton pump inhibitors (for example omeprazole, esomeprazole, lansoprazole, pantoprazole); metformin and glicazide or glipizide or sitagliptin, baclofen and proton pump inhibitors.

Still more preferably the tablets of the invention contain a combination of a non steroidal anti-inflammatory agent with an A2d ligand as described in WO 2008/077599.

Particularly preferred is the combination of flurbiprofen and gabapentin.

The excipients used for the formulation of the layers of the tablet of the present invention are excipients conventionally used in pharmaceutical technique.

Layer A consists of a hydrophilic matrix containing a hydrophilic polymer able to swell in the presence of water and a substance able to develop gas in the gastric environment functional to the floating of the layer. The gas is entrapped into the gel which is formed following the swelling of the polymer of the hydrophilic matrix allowing layer A, once separated from the others layers, to float in the aqueous medium. In addition to the drug, to the hydrophilic polymer and to the gas developing substance, layer A generally contains also further excipients to allow the granulation of the active ingredient and then the tabletting.

The hydrophilic matrix generally consists of a swellable polymer of the class of cellulose derivatives such as hydroxypropylmethylcellulose, hydroxypropylcellulose and hydroxyethylcellulose. Hydroxypropylmethylcellulose is particularly preferred. Also alginates, scleroglucans, carragenans or non ionic polymers such as polyethylenoxide can be used.

Among the commercially available polyethyleneoxides which can be used in the tablet object of the present invention, PolyoxWSR 303 (viscosity range 1% sol. 7500-10000 cP), PolyoxWSR N12K (viscosity range 2% sol. 400-800 cP) and PolyoxWSR 301 (viscosity range 1% sol. 1650-5500 cP) can be cited.

The substances able to develop gas are generally carbonates or bicarbonates, preferably of alkali or alkaline-earth metals such as, for example, sodium, potassium, calcium, magnesium carbonate or bicarbonates and the like.

It is particularly preferred sodium bicarbonate which is generally present in amounts from 5% to 10% by weight, preferably from 7% to 10% by weight.

Layer B contains a disintegrating agent, preferably a super-disintegrating agent, or mixtures thereof to achieve a rapid disintegration in contact with an aqueous medium. In addition to the optionally present drug and to the disintegrating agent, layer B generally contains also further excipients of conventional use to allow granulation and then tabletting.

Among these excipients particularly preferred is calcium hydrogen phosphate which is generally present in amounts from 30% to 60% by weight, preferably from 41% to 48% by weight.

Specific examples of disintegrating agents are: starch, microcrystalline cellulose, combinations of sodium bicarbonate and citric/tartaric acid.

Specific examples of super-disintegrating agents are: crospovidone, sodium croscarmellose, sodium carboxymethyl starch.

Layer C consists of a gastro-resistant or delayed release or, more preferably, gastro-resistant and delayed release hydrophilic matrix. After splitting from the other layers, layer C passes practically unchanged through the stomach and reaches the intestine where it releases the drug.

In its preferred embodiment, the matrix of layer C consists of excipients of conventional use suitably selected to obtain a gastro-resistant formulation which does not need any protective film coating. The possibility to obtain a gastro-resistant matrix can be achieved by combining an anionic polymer, with poor solubility in acid medium, with a non ionic polymer which remarkably gels also at acid pH values. In this way, the gelifying polymer rapidly builds a gel barrier which slows down the entry of acid within the matrix. This latter remains very compact because of the presence of a negatively charged polymer which does not jell in the acid medium. This polymer brings to the disintegration of the matrix when the pH of the external environment moves to values above 5.0. Furthermore, the matrix contains an oligomer able to complex the drug molecules optionally dissolved during the jelling of the non-ionic polymer, preventing their diffusion to the external environment.

Preferred examples of said excipients, used alone or in combination, are methacrylic polymers soluble at basic pH, cellulose acetophthalate, hydroxypropylmethylcellulose, hydroxypropylmethylcellulose phthalate, sodium alginate, scleroglucan, carragenane or other anionic polymers.

Among the polymers which can be used in layer C of the trilayered tablet according to the present invention hydroxyproylmethylcellulose, sodium alginate and mixtures thereof are particularly preferred.

The amount of hydroxypropylmethylcellulose is generally from 18% to 40% by weight, preferably from 22% to 33% by weight.

The amount of sodium alginate is generally from 7% to 25% by weight, preferably from 11% to 22% by weight.

Among the complexing oligomers β-cyclodextrin in amounts generally from 7% to 25% by weight, preferably from 10% to 22% by weight, is preferably used.

For the gastro-resistant layer of the tablet object of the present invention, the combination sodium alginate, hydroxypropylmethylcellulose and β-cyclodextrin is particularly preferred.

The trilayered formulation object of the present invention is particularly suitable for the administration of combinations of active ingredients with different pharmacokinetic properties and/or dosage schedules.

In a particularly preferred embodiment, the trilayered tablet of the invention is used for the administration of a combination of gabapentin and flurbiprofenin a single oral pharmaceutical dosage form.

The gabapentin-flurbiprofen combination is particularly effective for the treatment of low urinary tract disorders with the following dosage regimen:
Gabapentin 375 mg, in two aliquots of 300 mg + 75 mg splitted among layer A and layer B respectively, and flurbiprofen 37.5 mg in layer C, twice a day.

The release kinetics of the two active ingredients from the pharmaceutical dosage form must be then able to cover a period of 6-12 hours between an administration and the subsequent one.

This is achieved by the trilayered tablet object of the present invention as follows:
layer A contains gabapentin (300 mg). After splitting from the other layers, this layer floats into the stomach and releases the second aliquot of gabapentin for a period of 6-8 hours;
layer B contains gabapentin (75 mg) and rapidly disintegrates into the stomach causing the splitting of the tablet into the two layers A and C and the immediate release of the first aliquot of gabapentin into the stomach;
layer C contains flurbiprofen (37.5 mg). The release of flurbiprofen starts in a significant way only when the layer reaches the intestine and continues for 6-8 hours.

In order to adapt the dosage regimen to different kinds of patients, it is also possible to prepare trilayered gabapentin/flurbiprofen tablets having a reduced dosage:
Gabapentin 250 mg, in two aliquots of 200 mg + 50 mg, splitted between layer A and layer B respectively, and flurbiprofen 25 mg in layer C.

As already described, the release kinetics of the two active ingredients from the pharmaceutical trilayered form must be able to cover a period of 6-12 hours between an administration and the subsequent one.

Layer A contains gabapentin (200 mg). After splitting from the other layers, this layer floats into the stomach and releases the second aliquot of gabapentin for a period of 6-8 hours.

Layer B contains gabapentin (50 mg) and rapidly disintegrates into the stomach causing the splitting of the tablet into the two layers A and C and the immediate release of the first aliquot of gabapentin into the stomach.

Layer C contains flurbiprofen (25 mg). The release of flurbiprofen starts only when the layer reaches the intestine and continues for 6-8 hours.

Another example of gabapentin/flurbiprofen trilayered tablets having a reduced dosage is:
Gabapentin 190 mg, in two aliquots of 150 mg + 40 mg, splitted between layer A and layer B respectively, and flurbiprofen 19 mg in layer C.

Also in this case, the release kinetics of the two active ingredients from the pharmaceutical trilayered form must be able to cover a period of 6-12 hours between an administration and the subsequent one.

Layer A contains gabapentin (150 mg). After splitting from the other layers, this layer floats into the stomach and releases the second aliquot of gabapentin for a period of 6-8 hours.

Layer B contains gabapentin (40 mg) and rapidly disintegrates into the stomach causing the splitting of the tablet into the two layers A and C and the immediate release of the first aliquot of gabapentin into the stomach.

Layer C contains flurbiprofen (19 mg). The release of flurbiprofen starts only when the layer reaches the intestine and continues for 6-8 hours.

Another dosage regimen which represents an help to the patient compliance is represented by the gabapentin/flurbiprofen trilayered tablets with increased dosage for the once-a-day administration:
Gabapentin 500 mg, in two aliquots of 450 mg + 50 mg, and flurbiprofen 50 mg in two aliquots of 45 mg and 5 mg.

Layer A contains gabapentin (450 mg). After splitting from the other layers, this layer floats into the stomach and releases the second aliquot of gabapentin.

Layer B contains gabapentin (50 mg) and flurbiprofen (5 mg) and rapidly disintegrates into the stomach causing the splitting of the tablet into the two layers A and C and the immediate release of the first aliquot of gabapentin and flurbiprofen into the stomach.

Layer C contains flurbiprofen (45 mg). The release of flurbiprofen starts only when the layer reaches the intestine.

The dissolution profile of the trilayered tablets according to the present invention has been evaluated by *in vitro* and *in vivo* experiments.

The *in vitro* experiments have been carried out in simulated gastric fluid also under conditions simulating unempty stomach. The resultant dissolution profiles did not show significant differences proving that the release of the drug from the tablets of the present invention was not affected by the content of the stomach.

The *in vivo* results confirm the release of gabapentin and flurbiprofen at different physiological sites, since the food prolonged the floating time of the gabapentin layer and it delayed the gastric transit time of the flurbiprofen layer, which releases the active ingredient at pH equal or greater than 4.5 when reaches duodenum.

### Brief description of the figures

Figure 1 - dissolution profile of an oblong trilayered tablets containing gabapentin and flurbiprofen, prepared as described in example 1
Figure 2A - dissolution profile of an oblong trilayered tablets containing gabapentin and flurbiprofen, prepared as described in example 2
Figure 2B - dissolution profile of gabapentin in a tablet prepared as described in example 2 in simulated gastric fluid at pH 1.2, pH 1.6 (fasting condition) and pH 5.0 (fed condition)
Figure 3 - dissolution profile of an oblong trilayered tablets containing gabapentin and flurbiprofen, prepared as described in example 3
Figure 4 - dissolution profile of a cylindrical trilayered tablets containing gabapentin and flurbiprofen, prepared as described in example 6
Figure 5 - dissolution profile of a cylindrical trilayered tablets containing gabapentin and flurbiprofen, prepared as described in example 7
   In order to better illustrate the present invention, without however limiting it, the following examples are now given.
Figure 6 - Mean plasma levels of gabapentin (±S.D.) in 24 healthy subjects in fasting and fed conditions.
Figure 7 - Mean plasma levels of flurbiprofen (±S.D.) in 24 healthy subjects in fasting and fed conditions.

### Example 1

### Oblong trilayered tablet containing gabapentin 375 mg and flurbiprofen 37.5 mg.

### Preparation of layer A

Hydroxypropylmethylcellulose and sodium bicarbonate were added to a granulate of gabapentin and glicerylbehenate, prepared by dry granulation. The powder was mixed in Turbula for 15 minutes. Subsequently talc, colloidal silica and magnesium stearate were added by mixing for further 5 minutes. The resultant mixture was compressed to obtain a layer weighing 458.33 mg.

The composition of layer A for each tablet is the following (within brackets percentage in weight):

| | |
|---|---|
| Gabapentin | 300 mg (65.46%) |
| Glicerylbehenate | 15 mg (3.27%) |
| Hydroxypropylmethylcellulose (Methocel K15M, Colorcon) | 94.5 mg (20.62%) |
| Sodium bicarbonate | 31.5 mg (6.88%) |
| Colloidal silica (Aerosil 200NF, Evonik) | 9.45 mg (2.06%) |
| Talc | 6.3 mg (1.37%) |
| Magnesium stearate | 1.58 mg (0.34%) |

### Preparation of layer B

Microcrystalline cellulose, calcium hydrogen phosphate dihydrate, crospovidone, starch, sodium croscarmellose and purple lake were added to a granulate of gabapentin and glicerylbehenate, prepared by dry granulation. The powder was mixed in Turbula for 15 minutes. Subsequently magnesium stearate and talc were added by mixing for further 5 minutes. The resultant mixture was compressed to obtain a layer weighing 236.06 mg.

The composition of layer B for each tablet is the following (within brackets percentage in weight):

| | |
|---|---|
| Gabapentin | 75 mg (31.77%) |
| Glicerylbehenate (Compritol 888 ATO, Gattefosse) | 3.75 mg (1.59%) |
| Microcrystalline cellulose (Avicel PH102, FMC) | 23.63 mg (10.01%) |
| Calcium hydrogen phosphate dihydrate | 114.98 mg (48.71%) |
| Crospovidone | 3.15 mg (1.33%) |
| Maize starch | 7.88 mg (3.34%) |
| Sodium croscarmellose | 3.94 mg (1.67%) |
| Talc | 3.15 mg (1.33%) |
| Magnesium stearate | 0.39 mg (0.16%) |
| Purple lake | 0.19 mg (0.08%) |

### Preparation of layer C

Sodium alginate, hydroxypropylmethylcellulose and β-cyclodextrin were added to a granulate of flurbiprofen, mannitol and polyvinylpyrrolidone, prepared by wet granulation. The powder was mixed in Turbula for 15 minutes. Subsequently magnesium stearate and yellow lake were added by mixing for further 5 minutes. The resultant mixture was compressed to obtain a layer weighing 200.2 mg.

The composition of layer C for each tablet is the following (within brackets percentage in weight):

| | |
|---|---|
| Flurbiprofen | 37.5 mg (18.73%) |
| Mannitol | 24.01 mg (12.0%) |
| Polyvinylpyrrolidone (PVPK30, BASF) | 4.72 mg (2.36%) |
| β-cyclodextrin | 44.16 mg (22.06%) |
| Sodium alginate | 44.16 mg (22.06%) |
| Hydroxypropylmethylcellulose (Methocel K4M, Colorcon) | 44.16 mg (22.06%) |
| Magnesium stearate | 1 mg (0.5%) |
| Yellow lake | 0.49 mg (0.24%) |

### Preparation of the trilavered tablets

The trilayered tablets were prepared by compressing in a single solid form layer A, layer B and layer C, prepared as above described, after having put them down in sequence into the die of a tabletting machine with oblong 9x19 mm punches.

The compression was carried out according to the following steps:
Step 1 - placing into the die the amount of mixture to be used for the preparation of layer A and pre-compressing up to a tap density value of about 1.43 g/ml.
Step 2 - placing the amount of mixture corresponding to layer B on the previous pre-compressed layer. The powder bed was pre-compressed again up to a tap density of about 1.26 g/ml for both layers.
Step 3 - placing layer C and final compression of the three layers to obtain a trilayered tablet having the following physical characterisitcs:
   thickness: 6.35 ± 0.03 mm
   hardness: 14.2 ± 0.8 Monsanto units

### Example 2

### Oblong trilavered tablet containing gabapentin 375 mg and flurbiprofen 37.5 mg

By working in a way similar to that described in example 1, trilayered tablets having the following composition of the layers for each tablet (between brackets the weight percentage for each layer) were prepared. Compared with example 1, the composition of layer C was changed to obtain a slower delayed flurbiprofen release.

### Composition of layer A

| | |
|---|---|
| Gabapentin | 300 mg (65.46%) |
| Glicerylbehenate (Compritol 888 ATO) | 15 mg (3.27%) |
| Hydroxypropylmethylcellulose (Methocel K15M) | 94.5 mg (20.62%) |
| Sodium bicarbonate | 31.5 mg (6.88%) |
| Colloidal silica (Aerosil 200NF) | 9.45 mg (2.06%) |
| Talc | 6.3 mg (1.37%) |
| Magnesium stearate | 1.58 mg (0.34%) |

### Composition of layer B

| | |
|---|---|
| Gabapentin | 75 mg (31.77%) |
| Glicerylbehenate (Compritol 888 ATO) | 3.75 mg (1.59%) |
| Microcrystalline cellulose (Avicel PH102) | 23.63 mg (10.01%) |
| Calcium hydrogen phosphate dihydrate | 114.98 mg (48.1%) |
| Crospovidone | 3.15 mg (1.33%) |
| Maize starch | 7.88 mg (3.34%) |
| Sodium croscarmellose | 3.94 mg (1.67%) |
| Talc | 3.15 mg (1.33%) |
| Magnesium stearate | 0.39 mg (0.16%) |
| Purple lake | 0.19 mg (0.08%) |

### Composition of layer C

| | |
|---|---|
| Flurbiprofen | 37.5 mg (18.73%) |
| Mannitol | 24.01 mg (12.0%) |
| Polyvinylpyrrolidone (PVPK30) | 4.72 mg (2.36%) |
| p-cyclodextrin | 44.16 mg (22.06%) |
| Sodium alginate | 22.08 mg (11.03%) |
| Hydroxypropylmethylcellulose (Methocel K4M) | 66.24 mg (33.09%) |
| Magnesium stearate | 1 mg (0.5%) |
| Yellow lake | 0.49 mg (0.24%) |
| The physical characteristics of the resultant trilayered tablets were the following: | |
| thickness: 6.45 ± 0.03 mm | |
| hardness: 11.5 ± 0.5 Monsanto units | |

### Example 3

### Oblong trilayered tablet containing gabapentin 375 mg and flurbiprofen 37.5 mg

By working in a way similar to that described in example 1, trilayered tablets having the following composition of the layers for each tablet (between brackets the weight percentage for each layer) were prepared. Compared with example 1, the composition of layer A was changed by replacing the hydrophilic polymer hydroxypropylmethylcellulose with polyethylenoxide.

### Composition of laver A

| | |
|---|---|
| Gabapentin | 300 mg (65.46%) |
| Glycerylbehenate (Compritol 888 ATO) | 15 mg (3.27%) |
| Polyethylenoxide (PolyoxWSR 303, Colorcon) | 94.5 mg (20.62%) |
| Sodium bicarbonate | 31.5 mg (6.88%) |
| Colloidal silica (Aerosil 200NF) | 9.45 mg (2.06%) |
| Talc | 6.3 mg (1.37%) |
| Magnesium stearate | 1.58 mg (0.34%) |

### Composition of layer B

| | |
|---|---|
| Gabapentin | 75 mg (31.77%) |
| Glycerylbehenate (Compritol 888 ATO) | 3.75 mg (1.59%) |
| Microcrystalline cellulose (Avicel PH102) | 23.63 mg (10.01%) |
| Calcium hydrogen phosphate dihydrate | 114.98 mg (48.71%) |
| Crospovidone | 3.15 mg (1.33%) |
| Maize starch | 7.88 mg (3.34%) |
| Sodium croscarmellose | 3.94 mg (1.67%) |
| Talc | 3.15 mg (1.33%) |
| Magnesium stearate | 0.39 mg (0.16%) |
| Purple lake | 0.19 mg (0.08%) |

### Composition of layer C

| | |
|---|---|
| Flurbiprofen | 37.5 mg (18.73%) |
| Mannitol | 24.01 mg (12.0%) |
| Polyvinylpyrrolidone (PVPK30) | 4.72 mg (2.36%) |
| p-cyclodextrin | 44.16 mg (22.06%) |
| Sodium alginate | 44.16 mg (22.06%) |
| Hydroxypropylmethylcellulose (Methocel K4M) | 44.16 mg (22.06%) |
| Magnesium stearate | 1 mg (0.5%) |
| Yellow lake | 0.49 mg (0.24%) |

The physical characteristics of the resultant trilayered tablets were the following:
thickness: 6.99 ± 0.02 mm
hardness: 13.8 ± 0.5 Monsanto units

### Example 4

### Oblong trilayered tablet containing gabapentin 375 mg and flurbiprofen 37.5 mg

By working in a way similar to that described in example 1, trilayered tablets having the following composition of the layers for each tablet (between brackets the weight percentage for each layer) were prepared. Compared with example 3, the composition of layer C was changed to obtain a slower delayed flurbiprofen release.

### Composition of layer A

| | |
|---|---|
| Gabapentin | 300 mg (65.46%) |
| Glycerylbehenate (Compritol 888 ATO) | 15 mg (3.27%) |
| Polyethylenoxide (PolyoxWSR 303, Colorcon) | 94.5 mg (20.62%) |
| Sodium bicarbonate | 31.5 mg (6.88%) |
| Colloidal silica (Aerosil 200NF) | 9.45 mg (2.06%) |
| Talc | 6.3 mg (1.37%) |
| Magnesium stearate | 1.58 mg (0.34%) |

### Composition of layer B

| | |
|---|---|
| Gabapentin | 75 mg (31.77%) |
| Glycerylbehenate (Compritol 888 ATO) | 3.75 mg (1.59%) |
| Microcrystalline cellulose (Avicel PH102) | 23.63 mg (10.01%) |
| Calcium hydrogen phosphate dihydrate | 114.98 mg (48.71%) |
| Crospovidone | 3.15 mg (1.33%) |
| Maize starch | 7.88 mg (3.34%) |
| Sodium croscarmellose | 3.94 mg (1.67%) |
| Talc | 3.15 mg (1.33%) |
| Magnesium stearate | 0.39 mg (0.16%) |
| Purple lake | 0.19 mg (0.08%) |

### Composition of layer C

| | |
|---|---|
| Flurbiprofen | 37.5 mg (18.73%) |
| Mannitol | 24.01 mg (12.0%) |
| Polyvinylpyrrolidone (PVPK30) | 4.72 mg (2.36%) |
| p-cyclodextrin | 44.16 mg (22.06%) |
| Sodium alginate | 22.08 mg (11.03%) |
| Hydroxypropylmethylcellulose (Methocel K4M) | 66.24 mg (33.09%) |
| Magnesium stearate | 1 mg (0.5%) |
| Yellow lake | 0.49 mg (0.24%) |

The physical characteristics of the resultant trilayered tablets were the following:
thickness: 6.55 ± 0.03 mm
hardness: 14.5 ± 0.5 Monsanto units

### Example 5

### Dissolution test

The dissolution profile of the trilayered tablets prepared as described in examples 1, 2 and 3 was determined by immersion into simulated gastric fluid (USP apparatus II for dissolution test).

After immersion, the tablets (n = 6) placed themselves on the bottom of the vessel and the development of CO₂ bubbles which remained entrapped into the hydroxypropylmethylcellulose gel formed in contact with the aqueous medium was observed from layer A. At the same time, layer B rapidly disintegrated in correspondence with the side surface of the layer exposed to the simulated gastric fluid.

The complete separation of the layers was observed after 54 seconds for the tablet prepared as described in example 1, after 57 seconds for the tablet prepared as described in example 2 and after 4 minutes for the tablet prepared as described in example 3.

The floating of layer A was observed about 24 seconds after the splitting of the layers for the tablet prepared as described in example 1; about simultaneously to the splitting of the layers for the tablet prepared as described in example 2 and about 2 minutes after the splitting of the layers for the tablet prepared as described in example 3.

The dissolution rate of trilayered tablets prepared as described in example 2, was evaluated also under simulated fasting and fed conditions. The evaluation was carried out under the following conditions: simulated gastric fluid at pH1.2 (SGF), simulated gastric fluid at pH 1.6 (FaSSGF) for fasting conditions and simulated gastric fluid at pH 5.0 (FeSSGFm) for fed conditions.

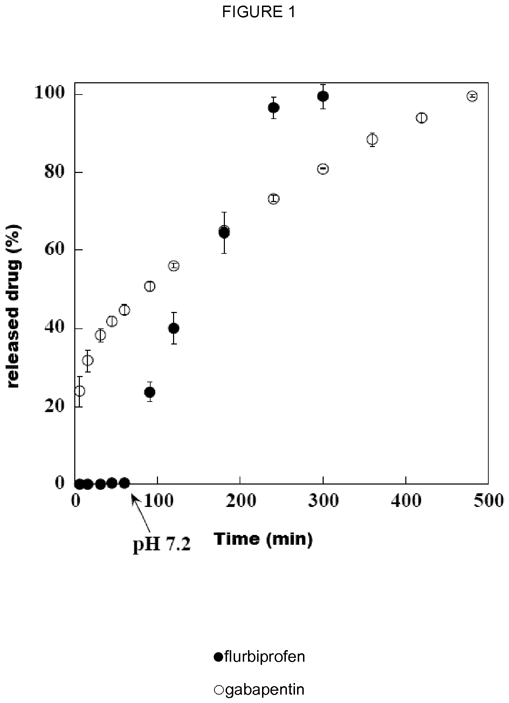

In Figure 1 the dissolution profile of gabapentin and flurbiprofen for the tablets of example 1 is graphically reported.

Gabapentin contained in layer B was released in the first 5 minutes while the remaining dose of gabapentin contained in layer A was slowly released in a prolonged way in about 8 hours. Flurbiprofen contained in layer C was not released in the first hour. After having raised the pH to 7.2 the release developed in a linear way reaching 100% in 5 hours.

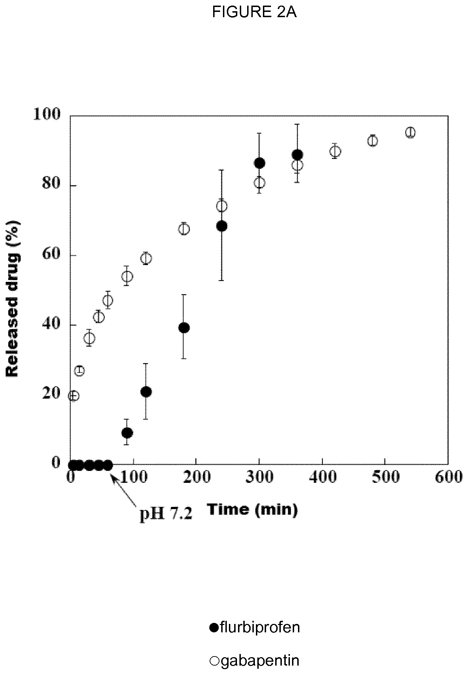

In Figure 2A the dissolution profile of gabapentin and flurbiprofen for the tablets of example 2 is graphically reported.

Gabapentin contained in layer B was released in the first 15 minutes while the remaining dose of gabapentin contained in layer A was slowly released in a prolonged way in about 9 hours. Flurbiprofen contained in layer C was not released at pH 1.2. After having raised the pH to 7.2 the release developed in a linear way reaching 90% in 6 hours.

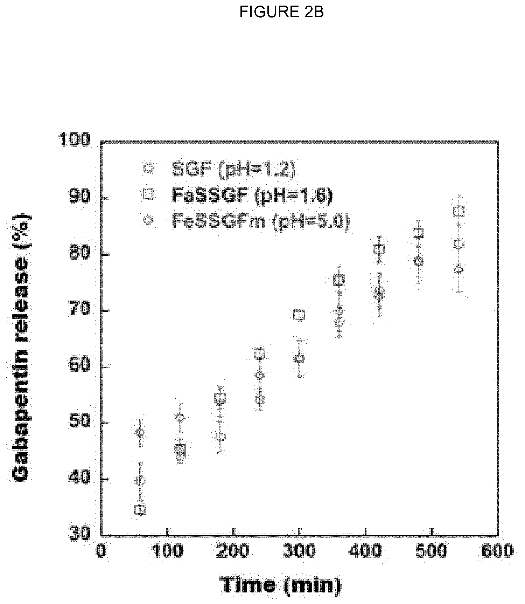

In Figure 2B the dissolution profiles of gabapentin for the tablets of example 2 in SGF, FaSSGF and FeSSGFm are graphically reported.

The three profiles did not show significant differences. The *f*₂ factor, similarity index between SGF and FaSSGF release profiles (*f*₂ = 83), between SGF and FeSSGFm release profiles (*f*₂ = 65.8) and between FaSSFG and FeSSGFm release profiles (*f*₂ = 55.7), was always higher than 50, showing that the drug release rate was not affected by the simulated conditions of fast or fed stomach.

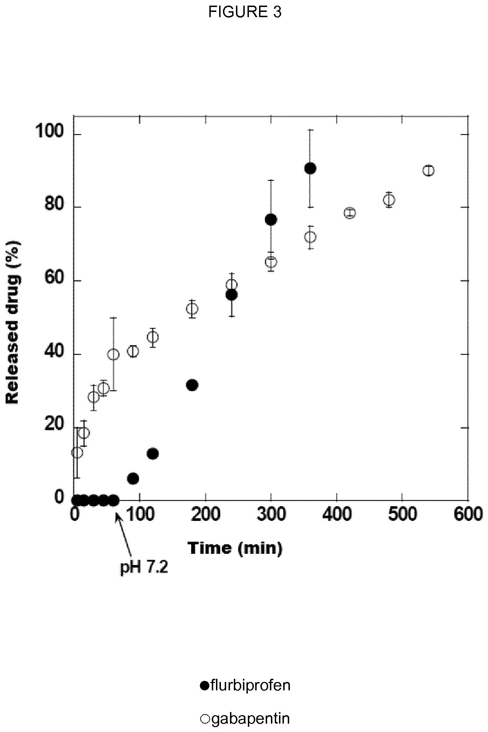

In Figure 3 the dissolution profile of gabapentin and flurbiprofen for the tablets of example 3 is graphically reported.

Gabapentin contained in layer B was released in the first 15 minutes while the remaining dose of gabapentin contained in layer A was slowly released in a prolonged way in about 9 hours. Flurbiprofen contained in layer C was not released at pH 1.2. After having raised the pH to 7.2 the release developed in a linear way reaching 90% in 6 hours.

### Example 6

### Cylindrical trilavered tablet containing gabapentin 375 mg and flurbiprofen 37.5 mg

By working in a way similar to that described in example 1, cylindrical trilayered tablets having the following composition of the layers for each tablet (between brackets the weight percentage for each layer) were prepared. Compared with example 1, the composition of layer A was changed by increasing the amount of sodium bicarbonate to promote the floating of the layer.

### Composition of layer A

| | |
|---|---|
| Gabapentin | 300 mg (63.28%) |
| Glycerylbehenate (Compritol 888 ATO) | 15 mg (3.16%) |
| Hydroxypropylmethylcellulose (Methocel K15M) | 94.5 mg (19.93%) |
| Sodium bicarbonate | 47.25 mg (9.97%) |
| Colloidal silica (Aerosil 200NF) | 9.45 mg (2.0%) |
| Talc | 6.3 mg (1.33%) |
| Magnesium stearate | 1.58 mg (0.33%) |

### Composition of layer B

| | |
|---|---|
| Gabapentin | 75 mg (31.77%) |
| Glycerylbehenate | 3.75 mg (1.59%) |
| Microcrystalline cellulose (Avicel PH102) | 23.63 mg (10.01%) |
| Calcium hydrogen phosphate dihydrate | 114.98 mg (48.71%) |
| Crospovidone | 3.15 mg (1.33%) |
| Maize starch | 7.88 mg (3.34%) |
| Sodium croscarmellose | 3.94 mg (1.67%) |
| Talc | 3.15 mg (1.33%) |
| Magnesium stearate | 0.39 mg (0.16%) |
| Purple lake | 0.19 mg (0.08%) |

### Composition of layer C

| | |
|---|---|
| Flurbiprofen | 37.5 mg (18.73%) |
| Mannitol | 24.01 mg (12.0%) |
| Polyvinylpyrrolidone (PVPK30) | 4.72 mg (2.36%) |
| β-cyclodextrin | 44.16 mg (22.06%) |
| Sodium alginate | 44.16 mg (22.06%) |
| Hydroxypropylcellulose (Methocel K4M) | 44.16 mg (22.06%) |
| Magnesium stearate | 1 mg (0.5%) |
| Yellow lake | 0.49 mg (0.24%) |

The compression was carried out with cylindrical 12 mm punches according to the following steps:
Step 1 - placing into the die the amount of mixture to be used for the preparation of layer A and pre-compressing up to a tap density value of about 1.1 g/ml.
Step 2 - placing the amount of mixture corresponding to layer B on the previous pre-compressed layer. The powder bed was pre-compressed again up to a tap density of about 1.39 g/ml for both layers.
Step 3 - placing layer C and final compression of the three layers to obtain a trilayered tablet having the following physical characteristics:
   thickness: 7.6 ± 0.08 mm
   hardness: 9.6 ± 1.7 Monsanto units

### Example 7

### Cylindrical trilayered tablet containing gabapentin 375 mg and flurbiprofen 37.5 mg

By working in a way similar to that described in example 6, cylindrical trilayered tablets having the following composition of the layers for each tablet (between brackets the weight percentage for each layer) were prepared. Compared with example 6, the composition of layer C was changed to obtain a slower flurbiprofen delayed release.

### Composition of layer A

| | |
|---|---|
| Gabapentin | 300 mg (63.28%) |
| Glycerylbehenate (Compritol 888 ATO) | 15 mg (3.16%) |
| Hydroxypropylmethylcellulose (Methocel K15M) | 94.5 mg (19.93%) |
| Sodium bicarbonate | 47.25 mg (9.97%) |
| Colloidal silica (Aerosil 200NF) | 9.45 mg (2.0%) |
| Talc | 6.3 mg (1.33%) |
| Magnesium stearate | 1.58 mg (0.33%) |

### Composition of layer B

| | |
|---|---|
| Gabapentin | 75 mg (31.77%) |
| Glycerylbehenate (Compritol 888 ATO) | 3.75 mg (1.59%) |
| Microcrystalline cellulose (Avicel PH102) | 23.63 mg (10.01%) |
| Calcium hydrogen phosphate dihydrate | 114.98 mg (48.71%) |
| Crospovidone | 3.15 mg (1.33%) |
| Maize starch | 7.88 mg (3.34%) |
| Sodium croscarmellose | 3.94 mg (1.67%) |
| Talc | 3.15 mg (1.33%) |
| Magnesium stearate | 0.39 mg (0.16%) |
| Purple lake | 0.19 mg (0.08%) |

### Composition of layer C

| | |
|---|---|
| Flurbiprofen | 37.5 mg (18.73%) |
| Mannitol | 24.01 mg (12.0%) |
| Polyvinylpyrrolidone (PVPK30) | 4.72 mg (2.36%) |
| β-cyclodextrin | 44.16 mg (22.06%) |
| Sodium alginate | 22.08 mg (11.03%) |
| Hydroxypropylmethylcellulose (Methocel K4M) | 66.24 mg (33.09%) |
| Magnesium stearate | 1 mg (0.5%) |
| Yellow lake | 0.49 mg (0.24%) |

The physical characteristics of the resultant trilayered tablets were the following:
thickness: 7.6 ± 0.03 mm
hardness: 12.5 ± 0.5 Monsanto units

### Example 8

### Dissolution test

The dissolution profile of the trilayered tablets prepared as described in examples 6, and 7 was determined by immersion into simulated gastric fluid (USP apparatus II for dissolution test).

After immersion, the tablets (n = 6) placed themselves on the bottom of the vessel and the development of CO₂ bubbles which remained entrapped into the hydroxypropylmethylcellulose gel formed in contact with the aqueous medium was observed from layer A. At the same time, layer B rapidly disintegrated in correspondence with the side surface of the layer exposed to the simulated gastric fluid.

The beginning of the floating of layer A was observed about 29±4 seconds for the tablet prepared as described in example 4, about simultaneously to the splitting of the layers. For the tablet prepared as described in example 5, the beginning of the floating of layer A was observed after 24±8 seconds, also in this case about simultaneously to the splitting of the layers.

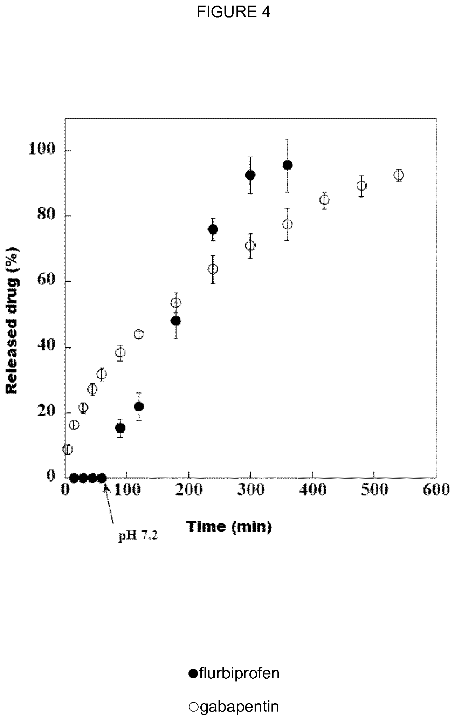

In Figure 4 the dissolution profile of gabapentin and flurbiprofen for the tablets of example 6 is graphically reported.

Gabapentin contained in layer B was released in the first 15 minutes while the remaining dose of gabapentin contained in layer A was slowly released in a prolonged way reaching 90% in about 9 hours. Flurbiprofen contained in layer C was not released in the first hour. After having raised the pH to 7.2 the release reached 100% in 5 hours.

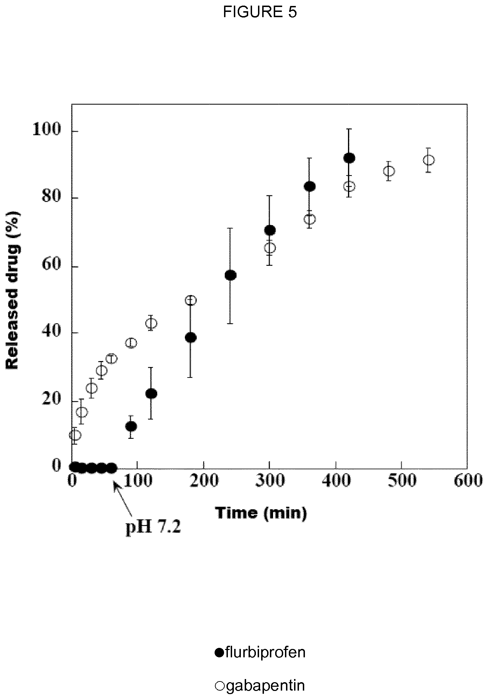

In Figure 5 the dissolution profile of gabapentin and flurbiprofen for the tablets of example 7 is graphically reported.

Gabapentin contained in layer B was released in the first 15 minutes while the remaining dose of gabapentin contained in layer A was slowly released in a prolonged way in about 9 hours. Flurbiprofen contained in layer C was not released at pH 1.2. After having raised the pH to 7.2 the release developed in a linear way reaching 90% in 7 hours.

### Example 9

### Oblong trilavered tablet containing gabapentin 250 mg and flurbiprofen 25 mg

By working in a way similar to that described in example 1, trilayered tablets with reduced dosage having the following composition of the layers for each tablet (between brackets the weight percentage for each layer) were prepared. Compared with the formulations containing 375 and 37.5 mg, the removed amounts of active ingredients were replaced by mannitol. In case of the floating prolonged release gabapentin layer (Layer A), the amount of gabapentin was replaced by an equal amount of mannitol dry granulated with glycerylbehenate. In case of the delayed release flurbiprofen layer, the removed amount of drug was replaced by an equivalent amount of mannitol in the granulate.

### Composition of layer A

| | |
|---|---|
| Gabapentin | 200 mg (43.64%) |
| Glycerylbehenate (Compritol 888 ATO) | 10 mg (2.18%) |
| Mannitol (Pearlitol 160C, Roquette) | 100 mg (21.82%) |
| Glycerylbehenate (Compritol 888 ATO) | 5 mg (1.09%) |
| Hydroxypropylmethylcellulose (Methocel K15M) | 94.5 mg (20.62%) |
| Sodium bicarbonate | 31.5 mg (6.87%) |
| Colloidal silica (Aerosil 200NF) | 9.45 mg (2.06%) |
| Talc | 3.15 mg (1.37%) |
| Magnesium stearate | 1.58 mg (0.35%) |

### Composition of layer B

| | |
|---|---|
| Gabapentin | 50 mg (21.18%) |
| Glycerylbehenate (Compritol 888 ATO) | 2.5 mg (1.06%) |
| Microcrystalline cellulose (Avicel PH102) | 29.02 mg (12.99%) |
| Calcium hydrogen phosphate dihydrate | 135.83 mg (57.54%) |
| Crospovidone | 3.15 mg (1.33%) |
| Maize starch | 7.86 mg (3.34%) |
| Sodium croscarmellose | 3.94 mg (1.67%) |
| Talc | 3.15 mg (1.33%) |
| Magnesium stearate | 0.39 mg (0.17%) |
| Purple lake | 0.19 mg (0.08%) |

### Composition of layer C

| | |
|---|---|
| Flurbiprofen | 25 mg (12.15%) |
| Mannitol (Pearlitol 160C) | 16 mg (7.77%) |
| Polyvinylpyrrolidone (PVPK30) | 4.4 mg (2.14%) |
| β-cyclodextrin | 45.4 mg (22.06%) |
| Sodium alginate | 45.4 mg (22.06%) |
| Hydroxypropylmethylcellulose (MethocelK4M) | 45.4 mg (22.06%) |
| Mannitol (Pearlitol SD200, Roquette) | 22.7 mg (11.03%) |
| Magnesium stearate | 1 mg (0.49%) |
| Yellow lake | 0.49 mg (0.24%) |

The physical characteristics of the resultant trilayered tablets were the following:
thickness: 6.29 ± 0.03 mm
hardness: 14.6 ± 0.7 Monsanto units

### Example 10

### Oblong trilayered tablet containing gabapentin 250 mg and flurbiprofen 25 mg

By working in a way similar to that described in example 9, trilayered tablets having the following composition of the layers for each tablet (between brackets the weight percentage for each layer) were prepared. Compared with example 9, the composition of layer C was changed to obtain a slower flurbiprofen delayed release.

### Composition of layer A

| | |
|---|---|
| Gabapentin | 200 mg (43.64%) |
| Glycerylbehenate (Compritol 888 ATO) | 10 mg (2.18%) |
| Mannitol (Pearlitol 160C) | 100 mg (21.82%) |
| Glycerylbehenate (Compritol 888 ATO) | 5 mg (1.09%) |
| Hydroxypropylmethylcellulose (Methocel K15M) | 94.5 mg (20.62%) |
| Sodium bicarbonate | 31.5 mg (6.87%) |
| Colloidal silica (Aerosil 200NF) | 9.45 mg (2.06%) |
| Talc | 3.15 mg (1.37%) |
| Magnesium stearate | 1.58 mg (0.35%) |

### Composition of layer B

| | |
|---|---|
| Gabapentin | 50 mg (21.18%) |
| Glycerylbehenate (Compritol 888 ATO) | 2.5 mg (1.06%) |
| Microcrystalline cellulose (Avicel PH102) | 29.02 mg (12.99%) |
| Calcium hydrogen phosphate dihydrate | 135.83 mg (57.54%) |
| Crospovidone | 3.15 mg (1.33%) |
| Maize starch | 7.86 mg (3.34%) |
| Sodium croscarmellose | 3.94 mg (1.67%) |
| Talc | 3.15 mg (1.33%) |
| Magnesium stearate | 0.39 mg (0.17%) |
| Purple lake | 0.19 mg (0.08%) |

### Composition of layer C

| | |
|---|---|
| Flurbiprofen | 25 mg (12.15%) |
| Mannitol (Pearlitol 160C) | 16 mg (7.77%) |
| Polyvinylpyrrolidone (PVPK30) | 4.4 mg (2.14%) |
| β-cyclodextrin | 45.4 mg (22.06%) |
| Sodium alginate | 22.7 mg (11.03%) |
| Hydroxypropylmethylcellulose (MethocelK4M) | 68.1 mg (33.09%) |
| Mannitol (Pearlitol SD200) | 22.7 mg (11.03%) |
| Magnesium stearate | 1 mg (0.49%) |
| Yellow lake | 0.49 mg (0.24%) |

The physical characteristics of the resultant trilayered tablets were the following:
thickness: 6.34 ± 0.09 mm
hardness: 16.0 ± 0.8 Monsanto units

### Example 11

### Oblong trilayered tablet containing gabapentin 190 mg and flurbiprofen 19 mg

By working in a way similar to that described in example 1, trilayered tablets with reduced dosage having the following composition of the layers for each tablet (between brackets the weight percentage for each layer) were prepared. Compared with the formulations containing 375 and 37.5 mg, the removed amounts of active ingredients were replaced by mannitol. In case of the floating prolonged release gabapentin layer (Layer A), the amount of gabapentin was replaced by an equal amount of mannitol dry granulated with glycerylbehenate. Also in the other two layers, the removed amount of drug was replaced by an equivalent amount of mannitol in the granulate.

### Composition of layer A

| | |
|---|---|
| Gabapentin | 150 mg (32.75%) |
| Mannitol (Pearlitol 160C) | 150 mg (32.75%) |
| Glycerylbehenate (Compritol 888 ATO) | 15 mg (3.28%) |
| Hydroxypropylmethylcellulose (Methocel K15M) | 94.5 mg (20.63%) |
| Sodium bicarbonate | 31.5 mg (6.88%) |
| Colloidal silica (Aerosil 200NF) | 9.12 mg (1.99%) |
| Talc | 6.3 mg (1.37%) |
| Magnesium stearate | 1.58 mg (0.35%) |

### Composition of layer B

| | |
|---|---|
| Gabapentin | 40 mg (14.29%) |
| Mannitol (Pearlitol 160C) | 40 mg (14.29%) |
| Glycerylbehenate (Compritol 888 ATO) | 4 mg (1.43%) |
| Microcrystalline cellulose (Avicel PH102) | 23.63 mg (8.44%) |
| Calcium hydrogen phosphate dihydrate | 114.98 mg (41.06%) |
| Crospovidone | 3.15 mg (1.12%) |
| Maize starch | 7.88 mg (2.81%) |
| Sodium croscarmellose | 3.94 mg (1.41%) |
| Mannitol (Pearlitol SD200, Roquette) | 37.04 mg (13.23%) |
| Colloidal silica (Aerosil 200NF) | 0.70 mg (0.25%) |
| Talc | 3.15 mg (1.12%) |
| Magnesium stearate | 1.40 mg (0.50%) |
| Purple lake | 0.13 mg (0.05%) |

### Composition of layer C

| | |
|---|---|
| Flurbiprofen | 19 mg (6.19%) |
| Mannitol (Pearlitol 160C) | 13.22 mg (4.3%) |
| Polyvinylpyrrolidone (PVPK30) | 1.34 mg (0.44%) |
| β-cyclodextrin | 68.09 mg (22.18%) |
| Sodium alginate | 68.09 mg (22.18%) |
| Hydroxypropylmethylcellulose (MethocelK4M) | 68.09 mg (22.18%) |
| Mannitol (Pearlitol SD200) | 67.26 mg (21.91%) |
| Magnesium stearate | 1.51 mg (0.49%) |
| Yellow lake | 0.40 mg (0.13%) |

The physical characteristics of the resultant trilayered tablets were the following:
thickness: 7.43 ± 0.07 mm
hardness: 12.4 ± 1.5 Monsanto units

### Example 12

### Oblong trilayered tablet containing gabapentin 190 mg and flurbiprofen 19 mg

By working in a way similar to that described in example 11, trilayered tablets having the following composition of the layers for each tablet (between brackets the weight percentage for each layer) were prepared. Compared with example 11, the composition of layer C was changed to obtain a slower flurbiprofen delayed release.

### Composition of layer A

| | |
|---|---|
| Gabapentin | 150 mg (32.75%) |
| Mannitol (Pearlitol 160C) | 150 mg (32.75%) |
| Glycerylbehenate | 15 mg (3.28%) |
| Hydroxypropylmethylcellulose (Methocel K15M) | 94.5 mg (20.63%) |
| Sodium bicarbonate | 31.5 mg (6.88%) |
| Colloidal silica (Aerosil 200NF) | 9.12 mg (1.99%) |
| Talc | 6.3 mg (1.37%) |
| Magnesium stearate | 1.58 mg (0.35%) |

### Composition of layer B

| | |
|---|---|
| Gabapentin | 40 mg (14.29%) |
| Mannitol (Pearlitol 160C) | 40 mg (14.29%) |
| Glycerylbehenate (Compritol 888 ATO) | 4 mg (1.43%) |
| Microcrystalline cellulose (Avicel PH102) | 23.63 mg (8.44%) |
| Calcium hydrogen phosphate dihydrate | 114.98 mg (41.06%) |
| Crospovidone | 3.15 mg (1.12%) |
| Maize starch | 7.88 mg (2.81%) |
| Sodium croscarmellose | 3.94 mg (1.41%) |
| Colloidal silica (Aerosil 200NF) | 0.70 mg (0.25%) |
| Mannitol (Pearlitol SD200) | 37.04 mg (13.23%) |
| Talc | 3.15 mg (1.12%) |
| | |
| Magnesium stearate | 1.40 mg (0.50%) |
| Purple lake | 0.13 mg (0.05%) |

### Composition of layer C

| | |
|---|---|
| Flurbiprofen | 19 mg (6.19%) |
| Mannitol (Pearlitol 160C) | 13.22 mg (4.3%) |
| Polyvinylpyrrolidone (PVPK30) | 1.34 mg (0.44%) |
| β-cyclodextrin | 68.09 mg (22.18%) |
| Sodium alginate | 34.05 mg (11.09%) |
| Hydroxypropylmethylcellulose (MethocelK4M) | 102.13 mg (32.27%) |
| Mannitol (Pearlitol SD200) | 67.26 mg (21.91%) |
| Magnesium stearate | 1.51 mg (0.49%) |
| Yellow lake | 0.40 mg (0.13%) |

The physical characteristics of the resultant trilayered tablets were the following:
thickness: 7.46 ± 0.06 mm
hardness: 11.8 ± 0.9 Monsanto units

### Example 13

### Oblong trilayered tablet containing gabapentin 500 mg and flurbiprofen 50 mg

By working in a way similar to that described in example 1, trilayered tablets with increased once-a-day dosage having the following composition of the layers for each tablet (between brackets the weight percentage for each layer) were prepared. Compared with the previous formulations the intermediate immediate release layer was prepared by mixing the two previously prepared granulates of gabapentin and flurbiprofen and by adding further excipients among which there is also β-cyclodextrin, needed to solubilize flurbiprofen in acid medium where the drug is very low soluble.

The fraction of flurbiprofen in the immediate release layer corresponds to 10% of the total dose.

### Composition of laver A

| | |
|---|---|
| Gabapentin | 450 mg (65.46%) |
| Glycerylbehenate (Compritol 888 ATO) | 22.48 mg (3.27%) |
| Hydroxypropylmethylcellulose (Methocel K15M) | 141.75 mg (20.62%) |
| Sodium bicarbonate | 47.3 mg (6.88%) |
| Colloidal silica (Aerosil 200NF) | 14.16 mg (2.06%) |
| Talc | 9.42 mg (1.37%) |
| Magnesium stearate | 2.34 mg (0.34%) |

### Composition of layer B

| | |
|---|---|
| Gabapentin | 50 mg (18.50%) |
| Flurbiprofen | 5 mg (1.85%) |
| Glycerylbehenate (Compritol 888 ATO) | 2.5 mg (0.92%) |
| Mannitol (Pearlitol 160C) | 3 mg (1.11%) |
| Polyvinylpyrrolidone (PVP K30) | 0.35 mg (0.13%) |
| β-cyclodextrin | 5 mg (1.85%) |
| Microcrystalline cellulose (Avicel PH102) | 26.63 mg (9.85%) |
| Calcium hydrogen phosphate dihydrate | 114.98 mg (42.54%) |
| Crospovidone | 3.15 mg (1.17%) |
| Maize starch | 7.88 mg (2.92%) |
| Sodium croscarmellose | 3.94 mg (1.46%) |
| Mannitol (Pearlitol SD200) | 42.49 mg (15.72%) |
| Colloidal silica (Aerosil 200NF) | 0.70 mg (0.26%) |
| Talc | 3.15 mg (1.17%) |
| Magnesium stearate | 1.40 mg (0.52%) |
| Purple lake | 0.13 mg (0.05%) |

### Composition of layer C

| | |
|---|---|
| Flurbiprofen | 45 mg (21.73%) |
| Mannitol (Pearlitol 160C) | 27 mg (13.04%) |
| Polyvinylpyrrolidone (PVPK30) | 3.15 mg (1.52%) |
| Mannitol (Pearlitol SD200) | 20 mg (9.66%) |
| β-cyclodextrin | 20 mg (9.66%) |
| Sodium alginate | 22.63 mg (10.93%) |
| Hydroxypropylmethylcellulose (MethocelK4M) | 67.89 mg (32.78%) |
| Magnesium stearate | 0.96 mg (0.46%) |
| Yellow lake | 0.48 mg (0.23%) |

The physical characteristics of the resultant trilayered tablets were the following:
thickness: 7.84 ± 0.07 mm
hardness: 12.5 ± 1.3 Monsanto units

### Example 14

### In vivo studies

A three layer tablet of gabapentin 375 mg and flurbiprofen 37.5 mg, prepared as described in example 4, was administered to 24 healthy male and female subjects, 18-55 years old, in fasting conditions and after a high fat meal in single dose according to a randomized cross-over design. The treatment periods were separated by a wash out interval of at least 5 days.

Each dose was taken with 240 mL of water in fasting conditions from at least 10 hours or 30 minutes after a standardized meal, which was high-caloric (approximately 1000 Kcal) and high-fat (approximately 50 percent of total caloric content of the meal).

Gabapentin and flurbiprofen plasma levels were determined by a validated HPLC/MS/MS analytical method at the following times: pre-dose, 0.5, 1, 1.5, 2, 3, 4, 5, 6, 7, 8, 10, 12, 16, 24 and 32 h post-dose.

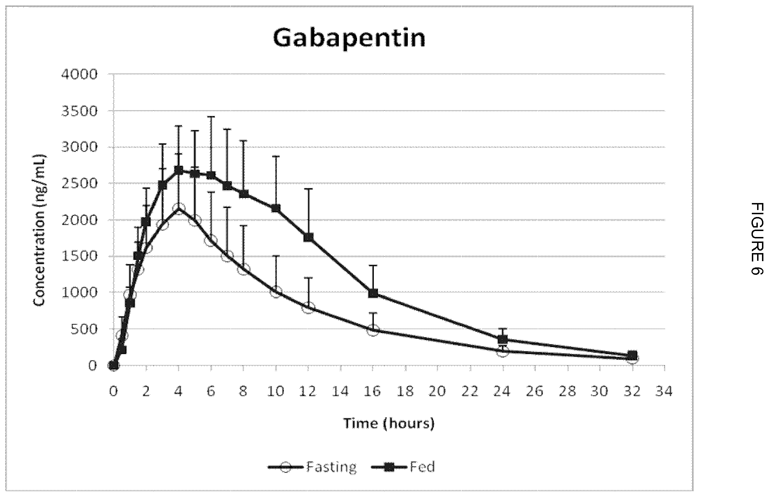

Results show that release of gabapentin from floating layer is prolonged by presence of food compared to the administration in fasting conditions; both Cmax and AUC were significantly increased (Tables 1 and 2). As shown in Figure 6 plasma gabapentin levels were maintained at plateau over 8 hours in fed conditions compared to about 4 hours in fasting conditions.

**Table 1**

| Main PK parameters (arithmetic mean ± s.d.) of gabapentin (n=24) | | |
|---|---|---|
| | Fasting conditions | Fed conditions |
| Cmax (ng/mL) | 2340 ± 748 | 3080 ± 727 |
| Tmax (hr)^ | 4 (2 - 7) | 5 (2 - 10) |
| AUCinf (hr*ng/mL) | 24126 ± 8150 | 39181 ± 9074 |

| | | |
|---|---|---|
| ^median (min - max) | | |

**Table 2**

| Statistical analysis on gabapentin | | | |
|---|---|---|---|
| Treament comparison | | Ratio of geometric means | |
| | Parameter | Ratio (%) | 90% Cl |
| Test / Reference (Fed / fasting) | Cmax | 136 % | 119 - 155 % |
| | AUCinf | 167 % | 150 - 187 % |

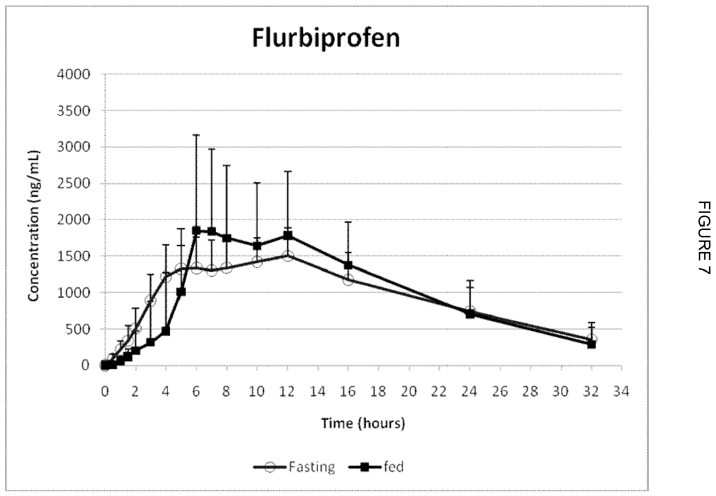

While results of flurbiprofen show that release was very low up to 2 hours after intake in fasting conditions and it was delayed up to 4 hours post dose in fed conditions, in agreement with a prolonged transit time through the stomach (Fig. 7). Plasma levels reached a plateau around 4 hour post dose in fasting conditions and around 6 hours in fed conditions, afterward levels were maintained constant up to 12 hours post dose. The high fat meal increased the Cmax compared to the fasting administration, however the AUC was not affected by food (Tables 3 and 4).

**Table 3**

| Main PK parameters (arithmetic mean ± s.d.) of flurbiprofen (n=24) | | |
|---|---|---|
| | Fasting conditions | Fed conditions |
| Cmax (ng/mL) | 1714 ± 380 | 2782 ± 943 |
| Tmax (hr)^ | 12 (4-24) | 10 (4 - 24) |
| AUCinf (hr*ng/mL) | 35725 ± 13162 | 36201 ± 14711 |

| | | |
|---|---|---|
| ^median (min - max) | | |

**Table 4**

| Statistical analysis on flurbiprofen data | | | |
|---|---|---|---|
| Treament comparison | | Ratio of geometric means | |
| | Parameter | Ratio (%) | 90% Cl |
| Test / Reference (Fed / fasting) | Cmax | 157 % | 136 - 181 % |
| | AUCinf | 98% | 94 - 103 % |

## Claims

1. A trilayered tablet for oral administration comprising a central rapidly disintegrating layer (layer B) placed between a swelling floating layer having a sustained release hydrophilic matrix (layer A) and a layer having a gastro-resistant or delayed release or gastro-resistant controlled release hydrophilic matrix (layer C).

2. A tablet according to claim 1 wherein layer A consists of a hydrophilic matrix swelling and floating in an aqueous medium and releasing the drug in a sustained way in the first tract of the gastro-intestinal apparatus.

3. A tablet according to claim 2 wherein the hydrophilic matrix contains a swelling polymer selected among cellulose derivatives, such as hydroxypropylmethylcellulose, hydroxypropylcellulose and hydroxyethylcellulose, alginates, scleroglucans, carrageenans and non ionic polymers, such as polyethylenoxide, and a substance able to develop gas selected among alkaline and alkaline earth metal carbonates and bicarbonates.

4. A tablet according to claim 3 wherein the substance able to develop gas is sodium bicarbonate in an amount from 5% to 10% by weight.

5. A tablet according to claim 1 wherein layer B is formulated to rapidly disintegrate so rapidly and immediately releasing the drug optionally contained therein into the stomach and causing the separation of the trilayered tablet into the two parts corresponding to layers A and C.

6. A tablet according to claim 5 wherein layer B contains a disintegrant selected among starch, microcrystalline cellulose, combinations of sodium bicarbonate and citric/tartaric acid, or a super-disintegrant selected among crospovidone, sodium croscarmellose, sodium carboxymethyl starch, or mixtures thereof.

7. A tablet according to claim 1 wherein layer C is a gastro-resistant controlled release formulation mainly releasing the drug in the intestine for a period of some hour.

8. A tablet according to claim 7 wherein the gastro-resistant formulation contains an excipient selected among methacrylic polymers soluble at basic pH, cellulose acetophthalate, hydroxypropylmethylcellulose, hydroxypropylmethylcellulose phthalate, sodium alginate, scleroglucan, carrageenan and other anionic polymers.

9. A tablet according to claim 7 wherein the gastro-resistant formulate contains a complexing oligomer.

10. A tablet according to claim 1 for the administration of a combination of two active ingredients selected among gabapentin, ACE-inhibitors, angiotensin II receptor antagonists, metformin, baclofen, H2-inhibitors, antibiotics for Helicobacter Pylori eradication, proton pump inhibitors, non steroidal anti-inflammatory agents, calcium antagonists.

11. A tablet according to claim 10 for the administration of a combination selected among combinations of proton pump inhibitors and H2 receptor antagonists, combinations of non steroidal anti-inflammatory agents and proton pump inhibitors or H2 receptor antagonists, combinations of ACE-inhibitors and calcium antagonists, combinations of angiotensin II receptor antagonists and calcium antagonists, combinations of ACE-inhibitors and angiotensin II receptor antagonists, combinations of antibiotics for Helicobacter Pylori eradication and proton pump inhibitors, combinations of metformin and glicazide or glipizide or sitagliptin, combinations of baclofen and proton pump inhibitors.

12. A tablet according to claim 11 for the administration of a combination of flurbiprofen and gabapentin.

## Patentansprüche

1. Dreischichtige Tablette für eine orale Verabreichung, umfassend eine mittlere, sich schnell zersetzende Schicht (Schicht B), die zwischen einer quellenden fließenden Schicht mit einer hydrophilen Matrix mit nachhaltiger Freisetzung (Schicht A) und einer Schicht, die eine magensaftresistente oder mit verzögerter Freisetzung oder magensaftresistente hydrophile Matrix mit kontrollierter Freisetzung (Schicht C) aufweist.

2. Tablette nach Anspruch 1, wobei die Schicht A aus einer hydrophilen Matrix besteht, die in einem wässrigen Medium quellt und fließt und das Medikament im ersten Trakt des Magen-Darm-Trakts nachhaltig freisetzt.

3. Tablette nach Anspruch 2, wobei die hydrophile Matrix ein Quellpolymer enthält, das unter Cellulosederivaten ausgewählt ist, wie z. B. Hydroxypropylmethylcellulose, Hydroxypropylcellulose und Hydroxyethylcellulose, Alginate, Scleroglucane, Carrageenane und nicht ionische Polymere, wie z. B. Polyethylenoxid und eine Substanz, die in der Lage ist, Gas zu entwickeln, ausgewählt unter alkalischen und Alkalimetall-Carbonaten und -Bicarbonaten.

4. Tablette nach Anspruch 3, wobei die Substanz, die in der Lage ist, Gas zu entwickeln, Natriumbicarbonat in einer Menge von 5 bis 10 Gew.-% ist.

5. Tablette nach Anspruch 1, wobei Schicht B formuliert ist, um sich schnell zu zersetzen, um so das Medikament, das optional darin enthalten ist, schnell und sofort in den Magen freizusetzen, und um eine Trennung der dreischichtigen Tablette die zwei Teile zu veranlassen, die den Schichten A und C entsprechen.

6. Tablette nach Anspruch 5, wobei Schicht B ein Zerfallsmittel enthält, ausgewählt unter Stärke, mikrokristalliner Cellulose, Kombinationen aus Natriumbicarbonat und Zitronen-/Tartarsäure oder einem Super-Zerfallsmittel, ausgewählt unter Crospovidon, Natriumcroscarmellose, Natriumcarboxymethylstärke oder Gemischen davon.

7. Tablette nach Anspruch 1, wobei Schicht C eine magensaftresistente Formulierung mit kontrollierter Freisetzung ist, die das Medikament im Darm über einen Zeitraum von einigen Stunden freisetzt.

8. Tablette nach Anspruch 7, wobei die magensaftresistente Formulierung einen Exzipienten enthält, ausgewählt unter Methacrylpolymeren, die bei basischem pH löslich sind, Celluloseacetophthalat, Hydroxypropylmethylcellulose, Hydroxypropylmethylcellulosphthalat, Natriumalginat, Scleroglucan, Carrageenan und anderen anionischen Polymeren.

9. Tablette nach Anspruch 7, wobei die magensaftresistente Formulierung ein Komplexbildner-Oligomer enthält.

10. Tablette nach Anspruch 1 für die Verabreichung einer Kombination aus zwei aktiven Bestandteilen, ausgewählt unter Gabapentin, ACE-Hemmern, Rezeptorantagonisten von Angiotensin II, Metformin, Baclofen, H2-Hemmer, Antibiotika zum Auslöschen von Helicobacter Pylori, Protonpumpeninhibitoren, nicht steroidalen entzündungshemmenden Mitteln, Calcium-Antagonisten.

11. Tablette nach Anspruch 10 für die Verabreichung einer Kombination, ausgewählt unter Kombinationen aus Protonpumpeninhibitoren und H2-Rezeptorantagonisten, Kombinationen aus nicht steroidalen entzündungshemmenden Mitteln und Protonpumpeninhibitoren oder H2-Rezeptorantagonisten, Kombinationen aus ACE-Hemmern und Calcium-Antagonisten, Kombinationen aus Rezeptorantagonisten von Angiotensin II und Calcium-Antagonisten, Kombinationen aus ACE-Hemmern und Rezeptorantagonisten von Angiotensin II, Kombinationen aus Antibiotika zum Auslöschen von Helicobacter Pylori und Protonpumpeninhibitoren, Kombinationen aus Metformin und Glicazid oder Glipizid oder Sitagliptin, Kombinationen aus Baclofen und Protonpumpeninhibitoren.

12. Tablette nach Anspruch 11 für die Verabreichung einer Kombination aus Flurbiprofen und Gabapentin.

## Revendications

1. Comprimé à trois couches pour administration orale comprenant une couche centrale à désintégration rapide (couche B) se trouvant entre une couche flottante gonflante comportant une matrice hydrophile à libération prolongée (couche A) et une couche présentant une matrice hydrophile gastro-résistante ou à libération retardée ou à libération contrôlée gastro-résistante (couche C).

2. Comprimé selon la revendication 1, ladite couche A se composant d'une matrice hydrophile gonflant et flottant en milieu aqueux et libérant le médicament de façon prolongée dans le premier tractus de l'appareil gastro-intestinal.

3. Comprimé selon la revendication 2, ladite matrice hydrophile contenant un polymère gonflant choisi parmi les dérivés de cellulose, tels que l'hydroxypropylméthylcellulose, l'hydroxypropylcellulose et l'hydroxyéthylcellulose, les alginates, les scléroglucanes, les carraghénanes et les polymères non ioniques, tels que l'oxyde de polyéthylène, et une substance capable de développer un gaz choisi les carbonates et les bicarbonates de métaux alcalins ou alcalino-terreux.

4. Comprimé selon la revendication 3, ladite substance capable de développer un gaz étant le bicarbonate de sodium en une quantité de 5 % à 10 % en poids.

5. Comprimé selon la revendication 1, ladite couche B étant formulée pour se désintégrer rapidement en libérant aussi rapidement et immédiatement le médicament éventuellement contenu en elle dans l'estomac et en provoquant la séparation du comprimé à trois couches en deux parties correspondant aux couches A et C.

6. Comprimé selon la revendication 5, ladite couche B contenant un délitant choisi parmi l'amidon, la cellulose microcristalline, des combinaisons de bicarbonate de sodium et d'acide citrique/tartrique, ou un super-délitant choisi parmi la crospovidone, le croscarmellose sodique, l'amidon carboxyméthylique sodique, ou des mélanges de ceux-ci.

7. Comprimé selon la revendication 1, ladite couche C étant une formulation à libération contrôlée gastro-résistante libérant principalement le médicament dans l'intestin pendant une période de quelques heures.

8. Comprimé selon la revendication 7, ladite formulation gastro-résistante contenant un excipient choisi parmi les polymères méthacryliques soluble à pH basique, l'acétophtalate de cellulose, l'hydroxypropylméthylcellulose, le phtalate d'hydroxypropylméthylcellulose, l'alginate de sodium, le scléroglucane, le carraghénane et les autres polymères anioniques.

9. Comprimé selon la revendication 7, ladite formulation gastro-résistante contenant un oligomère complexant.

10. Comprimé selon la revendication 1 pour l'administration d'une combinaison de deux principes actifs choisis parmi la gabapentine, les inhibiteurs e l'ECA, les antagonistes des récepteurs de l'angiotensine II, la metformine, le baclofène, les inhibiteurs des récepteurs H2, les antibiotiques destinés à l'éradication d'Helicobacter Pylori, les inhibiteurs de pompes à protons, les agents anti-inflammatoires non stéroïdiens, les antagonistes du calcium.

11. Comprimé selon la revendication 10 pour l'administration d'une combinaison choisi parmi les combinaisons d'inhibiteurs de pompes à protons et d'antagonistes des récepteurs H2, les combinaisons d'agents anti-inflammatoires non stéroïdiens et d'inhibiteurs de pompes à protons ou d'antagonistes des récepteurs H2, les combinaisons d'inhibiteurs de l'ECA et d'antagonistes du calcium, les combinaisons d'antagonistes des récepteurs de l'angiotensine II et d'antagonistes du calcium, les combinaisons d'inhibiteurs de l'ECA et d'antagonistes des récepteurs de l'angiotensine II, les combinaisons d'antibiotiques destinés à l'éradication d'Helicobacter Pylori et d'inhibiteurs de pompes à protons, des combinaisons de metformine et de glicazide ou de glipizide ou de sitagliptine, des combinaisons de baclofène et d'inhibiteurs de pompes à protons.

12. Comprimé selon la revendication 11 pour l'administration d'une combinaison de flurbiprofène et de gabapentine.
